# EUROPEAN PATENT SPECIFICATION

(11) **EP 4 404 846 B1**
(45) Date of publication and mention of the grant of the patent: **08.10.2025**
(21) Application number: 22769256.3
(22) Date of filing: 26.08.2022
(51) Int. Cl.: A61B 8/08, A61B 8/06

(54) **METHODS AND SYSTEMS FOR GENERATING LIKELIHOOD OF HEART FAILURE WITH PRESERVED EJECTION FRACTION (HFPEF)**
VERFAHREN UND SYSTEME ZUR ERZEUGUNG DER WAHRSCHEINLICHKEIT EINES HERZVERSAGENS MIT ERHALTENER EJEKTIONSFRAKTION (HFPEF)
PROCÉDÉS ET SYSTÈMES POUR GÉNÉRER LA PROBABILITÉ D'UNE INSUFFISANCE CARDIAQUE À FRACTION D'ÉJECTION PRÉSERVÉE (ICFEP)

(30) Priority: 24.09.2021 US 202163247823 P; 14.10.2021 EP 21202598
(43) Date of publication of application: 31.07.2024
(73) Proprietor: Koninklijke Philips N.V., 5656 AG Eindhoven (NL)
(72) Inventor: SADEGHI, Seyedali, 5656 AG Eindhoven (NL); RAFTER, Patrick, Gabriels, 5656AG Eindhoven (NL)
(74) Representative: Philips Intellectual Property & Standards
(86) International application number: PCT/EP2022/073836
(87) International publication number: WO 2023/046413

(56) References cited:
- WO-A1-2006/046982
- US-A1- 2004 059 220
- NOBUYUKI KAGIYAMA ET AL: "Machine Learning Assessment of Left Ventricular Diastolic Function Based on Electrocardiographic Features", JOURNAL OF THE AMERICAN COLLEGE OF CARDIOLOGY, vol. 76, no. 8, 1 August 2020 (2020-08-01), AMSTERDAM, NL, pages 930 - 941, XP055753720, ISSN: 0735-1097, DOI: 10.1016/j.jacc.2020.06.061

## Description

### FIELD OF THE INVENTION

The present disclosure is directed generally to methods and systems for providing a visualized likelihood of heart failure with preserved ejection fraction for a subject.

### BACKGROUND OF THE INVENTION

Heart failure, which can be defined as the inability of the heart to provide sufficient cardiac output while maintaining normal filling pressures, affects at least 26 million people worldwide and is estimated to increase by 46% by 2030. Two types of heart failure exist: (1) heart failure with reduced ejection fraction (HFrEF) and (2) heart failure with preserved ejection fraction (HFpEF). The latter, HFpEF, makes up 50% of heart failure cases and is characterized by impaired relaxation of the left ventricle (LV) during diastole and increased filling pressures caused by altered LV mechanical properties, most notably higher stiffness. Conditions such as cardiac amyloidosis, coronary artery disease, valvular disease, hypertrophic cardiomyopathy (HCM), pericardial disease, and hypertension can produce HFpEF.

Echocardiography is the primary imaging modality for HFpEF. However, HFpEF cannot be directly used for differential diagnosis. While there are current guidelines for the diagnosis of HFpEF, including history and physical examination, echocardiography, and cardiac catheterization if necessary, these guidelines are complicated and rarely followed. Even with these guidelines, identifying the root cause of HFpEF remains challenging.

As an example, cardiac amyloidosis is one of the most rapidly progressive forms of HFpEF, with a median survival from diagnosis, if untreated, ranging from <6 months for light chain amyloidosis (AL) to 3 to 5 years for transthyretin amyloidosis (ATTR). Currently, a definite diagnosis of ATTR amyloidosis is performed using Tc-99m-PYP/DPD/HMDP imaging. Echocardiography is usually the first test performed in patients presenting with heart failure. However, typical echocardiographic features of CA are most prominent in advanced disease and may be missed in earlier diseases, even when severe enough to cause heart failure. Particularly in early disease, echocardiography lacks specificity to precisely distinguish amyloid from non-amyloid infiltrative or hypertrophic heart diseases such as left ventricular hypertrophy (LVH). Therefore, ultrasound does not currently play a role in the HFpEF differential diagnosis, especially in the earlier stages.

There is agreement in the cardiac imaging community that there is a strong need for standardization of HFpEF differential diagnosis in order to improve the efficiency and efficacy of care and lead to better patient outcomes. Currently, there is no well-accepted and intelligent patient-specific method for predicting the likelihood of different HFpEF etiologies given the upstream patient's clinical context across different institutions. Therefore, there is a crucial need for establishing an intelligent data-driven decision support tool for HFpEF differential diagnosis in the world of cardiac care imaging. The document WO 2006/046982 A1 discloses diastolic function analysis based on ultrasound data and using a classifier for analyzing ultrasound images for diastolic function.

### SUMMARY OF THE INVENTION

Accordingly, there is a continued need for methods and systems for HFpEF differential diagnosis. Various embodiments and implementations herein are directed to a method and system configured to generate and present a visualized likelihood of HFpEF. A system, such as for example a patient analysis system, receives the result of an ultrasound analysis of the subject's heart, and extracts a plurality of ultrasound biomarkers for the patient from the received result of the ultrasound analysis. The system also receives a cardiac stiffness measurement for the subject's heart, and clinical information about the subject. The patient analysis system then analyzes, using a trained heart failure model configured to output a likelihood of at least one of a plurality of HFpEF etiologies, the extracted plurality of ultrasound biomarkers, the received cardiac stiffness measurement, and the received clinical information about the subject as input. The output of the trained model, which is the determined likelihood of at least one of a plurality of HFpEF etiologies, comprises a likelihood that the subject is experiencing heart failure with preserved ejection fraction. The system then displays a visualization of the generated likelihood of at least one of a plurality of HFpEF etiologies on a user interface of the system.

Generally, in one aspect, a method for providing a visualized likelihood of heart failure with preserved ejection fraction (HFpEF) for a subject is provided. The method includes: (i) receiving a result of an ultrasound analysis of the subject's heart from a current ultrasound exam; (ii) extracting, from the received result of the ultrasound analysis, a plurality of ultrasound biomarkers; (iii) receiving a cardiac stiffness measurement for the subject's heart from the current ultrasound exam and/or from a previous ultrasound exam; (iv) receiving clinical information about the subject; (v) analyzing, using a trained heart failure model, the extracted plurality of ultrasound biomarkers, the received cardiac stiffness measurement, and the received clinical information about the subject to generate a likelihood of at least one of a plurality of HFpEF etiologies, wherein the generated likelihood of at least one of a plurality of HFpEF etiologies comprises a likelihood that the subject is experiencing heart failure with preserved ejection fraction; and (vi) displaying, on a user interface, a visualization of the generated likelihood of at least one of a plurality of HFpEF etiologies.

According to an embodiment, the plurality of HFpEF etiologies comprises cardiac amyloidosis, coronary artery disease, hypertension, pericardial disease, hypertrophic cardiomyopathy, and valvular disease.

According to an embodiment, the method further includes receiving results of one or more previous imaging analyses of the subject's heart, wherein the imaging analysis is ultrasound imaging or another imaging modality; and receiving one or more ultrasound biomarkers from a previous imaging analysis; wherein analyzing, using a trained heart failure model, further comprises the received one or more previous imaging analyses and/or the one or more ultrasound biomarkers from a previous imaging analysis.

According to an embodiment, displaying further comprises displaying, on the user interface, the following: (i) the subject's name; (ii) one or more details about the ultrasound analysis; (iii) a likelihood of each of each of a plurality of HFpEF etiologies; and (iv) a treatment recommendation.

According to an embodiment, the method further includes determining that one or more ultrasound biomarkers are missing from the extracted plurality of ultrasound biomarkers; generating a request for the one or more missing ultrasound biomarkers; and receiving, in response to the request, at least one of the one or more missing ultrasound biomarkers.

According to an embodiment, the plurality of ultrasound biomarkers comprises one or more of ejection fraction, global longitudinal strain, flow propagation velocity, mitral inflow velocity at early diastole, mitral inflow velocity at late diastole, mitral annular velocity at early diastole, mitral annular velocity at late diastole, left atrium volume index, left ventricle thickness, septum thickness, thickness of one or more valves, right ventricle thickness, relative wall thickness, tricuspid regurgitation velocity, and left ventricle mass index.

According to an embodiment, the clinical information about the subject comprises one or more of an ultrasound exam type, a reason for the ultrasound analysis, age of the subject, sex of the subject, body mass index of the subject, atrial fibrillation status or diagnosis, and coronary artery disease status or diagnosis.

According to an embodiment, cardiac stiffness is measured by an atrial kick method, a valve closure method, and/or a shear wave method.

According to a second aspect is a system for providing a visualized likelihood of heart failure with preserved ejection fraction (HFpEF) for a subject. The system includes a trained heart failure model; a processor configured to: (i) receive a result of an ultrasound analysis of the subject's heart from a current ultrasound exam; (ii) extract, from the received result of the ultrasound analysis, a plurality of ultrasound biomarkers; (iii) receive a cardiac stiffness measurement for the subject's heart from the current ultrasound exam and/or from a previous ultrasound exam; (iv) receive clinical information about the subject; (v) analyze, using the trained heart failure model, the extracted plurality of ultrasound biomarkers, the received cardiac stiffness measurement, and the received clinical information about the subject to generate a likelihood of at least one of a plurality of HFpEF etiologies, wherein the generated likelihood of at least one of a plurality of HFpEF etiologies comprises a likelihood that the subject is experiencing heart failure with preserved ejection fraction; and (vi) generate a visualization of the generated likelihood of at least one of a plurality of HFpEF etiologies; and a user interface configured to provide the generated likelihood of at least one of a plurality of HFpEF etiologies.

According to an embodiment, the user interface is further configured to display: (i) the subject's name; (ii) one or more details about the ultrasound analysis; (iii) a likelihood of each of each of a plurality of HFpEF etiologies; and (iv) a treatment recommendation.

According to an embodiment, the processor is further configured to: determine that one or more ultrasound biomarkers are missing from the extracted plurality of ultrasound biomarkers; (ii) generate a request for the one or more missing ultrasound biomarkers; and receive, in response to the request, at least one of the one or more missing ultrasound biomarkers.

According to a third aspect is a non-transitory computer-readable storage medium comprising computer program code instructions which, when executed by a processor, enables the processor to carry out a method including: (i) receiving a result of an ultrasound analysis of the subject's heart from a current ultrasound exam; (ii) extracting, from the received result of the ultrasound analysis, a plurality of ultrasound biomarkers; (iii) receiving a cardiac stiffness measurement for the subject's heart from the current ultrasound exam and/or from a previous ultrasound exam; (iv) receiving clinical information about the subject; (v) analyzing, using a trained heart failure model, the extracted plurality of ultrasound biomarkers, the received cardiac stiffness measurement, and the received clinical information about the subject to generate a likelihood of at least one of a plurality of HFpEF etiologies, wherein the generated likelihood of at least one of a plurality of HFpEF etiologies comprises a likelihood that the subject is experiencing heart failure with preserved ejection fraction; and (vi) displaying, on a user interface, a visualization of the generated likelihood of at least one of a plurality of HFpEF etiologies.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.
Fig. 1 is a flowchart of a method for providing a visualized likelihood of HFpEF for a subject, in accordance with an embodiment.
Fig. 2 is a schematic representation of a patient analysis system, in accordance with an embodiment.
Fig. 3 is a schematic representation of a cardiac stiffness measurement, in accordance with an embodiment.
Fig. 4 is a flowchart of input to and output from a trained heart failure model of a patient analysis system, in accordance with an embodiment.
Fig. 5 is a flowchart of a method for training a heart failure model of a patient analysis system, in accordance with an embodiment.
Fig. 6 is a schematic representation of a visualization of HFpEF, in accordance with an embodiment.

### DETAILED DESCRIPTION OF EMBODIMENTS

The present disclosure describes various embodiments of a system and method configured to generate and present a visualized likelihood of HFpEF for a subject. More generally, Applicant has recognized and appreciated that it would be beneficial to provide an intelligent data-driven decision support tool for HFpEF differential diagnosis. Accordingly, a patient analysis system receives the result of an ultrasound analysis of the subject's heart, and extracts a plurality of ultrasound biomarkers for the patient from the received result of the ultrasound analysis. The system also receives a cardiac stiffness measurement for the subject's heart, and clinical information about the subject. The patient analysis system then analyzes, using a trained heart failure model configured to output a likelihood of at least one of a plurality of HFpEF etiologies, the extracted plurality of ultrasound biomarkers, the received cardiac stiffness measurement, and the received clinical information about the subject as input. The output of the trained model, which is the determined likelihood of at least one of a plurality of HFpEF etiologies, comprises a likelihood that the subject is experiencing heart failure with preserved ejection fraction. The system then displays a visualization of the generated likelihood of at least one of a plurality of HFpEF etiologies on a user interface of the system. The visualization of the generated likelihood can then be utilized by a healthcare professional to implement a healthcare treatment for the subject.

According to an embodiment, the systems and methods described or otherwise envisioned herein can, in some non-limiting embodiments, be implemented as an element for a commercial product for ultrasound imaging or analysis, or as an element for a commercial product for cardiovascular analysis such as Philips^{®} IntelliSpace Cardiovascular (ISCV) (available from Koninklijke Philips NV, the Netherlands), or as an element for a commercial product for patient analysis or monitoring, such as the Philips Patient Flow Capacity Suite (PFCS), or any suitable system.

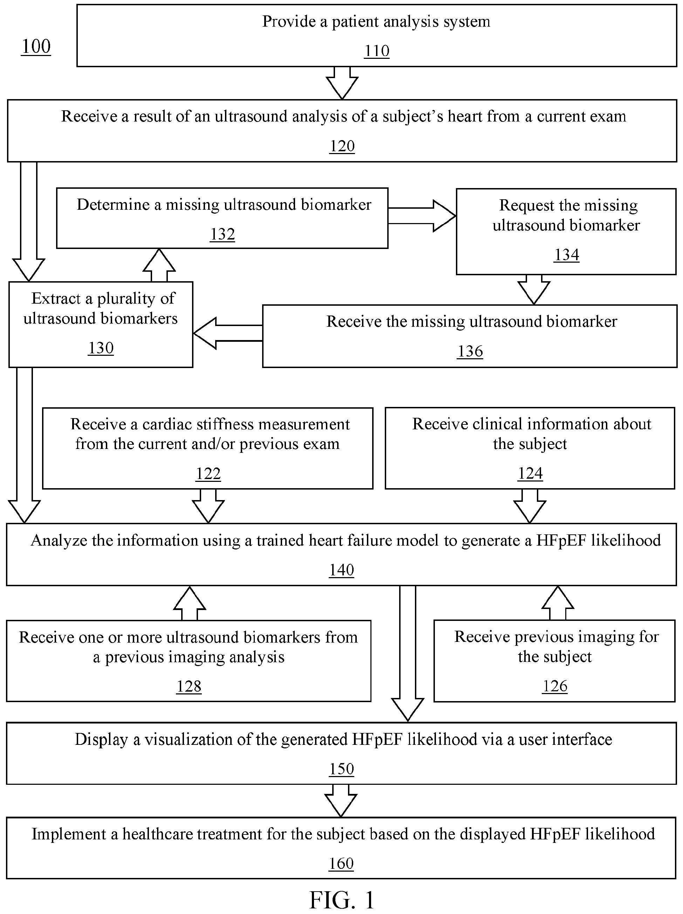

Referring to Fig. 1, in one embodiment is a flowchart of a method 100 for analyzing or determining a patient's likelihood of HFpEF using a patient analysis system. The methods described in connection with the figures are provided as examples only, and shall be understood not limit the scope of the disclosure. The patient analysis system can be any of the systems described or otherwise envisioned herein. The patient analysis system can be a single system or multiple different systems.

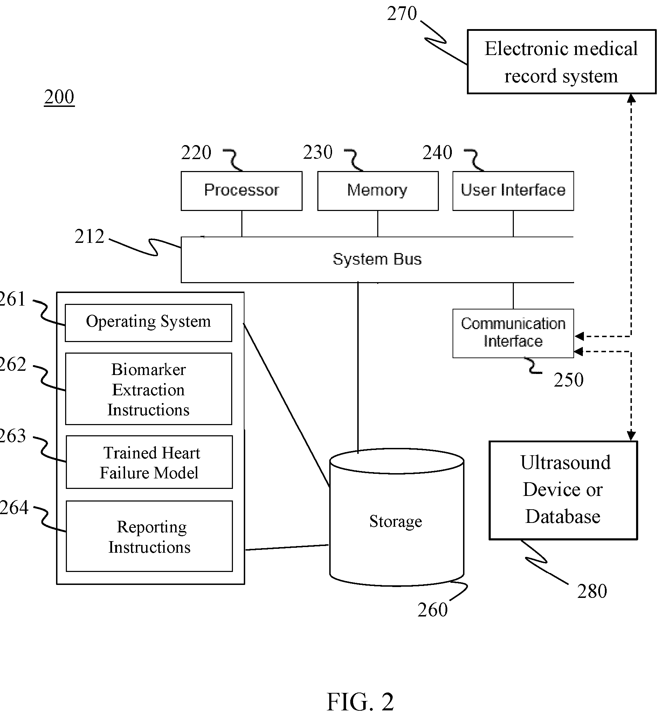

At step 110 of the method, a patient analysis system 200 is provided. Referring to an embodiment of a patient analysis system 200 as depicted in Fig. 2, for example, the system comprises one or more of a processor 220, memory 230, user interface 240, communications interface 250, and storage 260, interconnected via one or more system buses 212. It will be understood that Fig. 2 constitutes, in some respects, an abstraction and that the actual organization of the components of the system 200 may be different and more complex than illustrated. Additionally, patient analysis system 200 can be any of the systems described or otherwise envisioned herein. Other elements and components of patient analysis system 200 are disclosed and/or envisioned elsewhere herein.

At step 120 of the method, the patient analysis system receives a result of an ultrasound analysis of the subject's heart. The ultrasound analysis of the subject's heart can be any analysis sufficient to provide ultrasound data about the subject that can be utilized in downstream steps of the method. The ultrasound analysis of the subject's heart can be obtained using any ultrasound methodology or device capable of providing the ultrasound data utilized in downstream steps of the method. According to an embodiment, the ultrasound analysis of the subject's heart comprises a plurality of images obtained by an ultrasound device, and/or comprises a summary of or report for the plurality of images.

According to an embodiment, patient analysis system 200 comprises an ultrasound device capable of acquiring the required ultrasound images or analysis. According to another embodiment, patient analysis system 200 is in wired and/or wireless communication with a local or remote ultrasound device capable of acquiring the required ultrasound images or analysis. According to another embodiment, patient analysis system 200 is in wired and/or wireless communication with a local or remote database which stores the ultrasound images or analysis. The patient analysis system 200 can obtain the required ultrasound images or analysis from one or more of these sources.

According to an embodiment, the ultrasound analysis of the subject's heart is obtained by an ultrasound imaging specialist as part of a routine analysis of the subject or in response to possible or known medical issues being experienced by the subject. The ultrasound analysis may be performed or obtained for immediate or near-term analysis by the methods and systems described or otherwise envisioned herein, or may be performed or obtained for a future analysis by the methods and systems described or otherwise envisioned herein.

According to an embodiment, the ultrasound analysis of the subject's heart comprises 2D images or recording, 3D images or recording, and/or both.

At step 130 of the method, the patient analysis system extracts a plurality of ultrasound biomarkers from the received result of the ultrasound analysis. The plurality of ultrasound biomarkers can be any metrics, measurements, parameters, or other data extracted from an ultrasound analysis. According to an embodiment, the plurality of ultrasound biomarkers are quantitative parameters extracted from the received result of the ultrasound analysis or otherwise obtained during an ultrasound analysis. The ultrasound biomarkers can be extracted using any of a wide variety of methods for data extraction from ultrasound imaging. According to an embodiment, one or more of the plurality of ultrasound biomarkers are obtained or extracted automatically by software or algorithm associated with the ultrasound device or the patient analysis system. For example, one or more of the plurality of ultrasound biomarkers can be obtained with an ultrasound workstation solution. According to another embodiment, one or more of the plurality of ultrasound biomarkers are obtained or extracted manually during the ultrasound analysis.

According to an embodiment, the plurality of ultrasound biomarkers comprises one or more of ejection fraction, global longitudinal strain, flow propagation velocity, mitral inflow velocity at early diastole, mitral inflow velocity at late diastole, mitral annular velocity at early diastole, mitral annular velocity at late diastole, left atrium volume index, left ventricle thickness, septum thickness, thickness of one or more valves, right ventricle thickness, relative wall thickness, tricuspid regurgitation velocity, and left ventricle mass index. However, many other ultrasound biomarkers are possible.

The plurality of ultrasound biomarkers received by or extracted by the patient analysis system can be utilized, before or after data processing, immediately or may be stored in local or remote storage for use in further steps of the method.

According to an embodiment, one or more ultrasound biomarkers may be missing from the plurality of ultrasound biomarkers. Accordingly, at optional step 132 of the method, the system determines that one or more ultrasound biomarkers are missing from the received or extracted plurality of ultrasound biomarkers. According to an embodiment, the system can comprise a list of required minimum ultrasound biomarkers required for downstream analysis by the method, and can analyze a set of extracted or received ultrasound biomarkers to determine whether each of the required minimum ultrasound biomarkers required for downstream analysis by the method is present in the set. Accordingly, the system may determine that all required ultrasound biomarkers are present, or may determine that one or more required ultrasound biomarkers are missing from the received or extracted set of ultrasound biomarkers.

At optional step 134 of the method, the system generates a request for the one or more missing ultrasound biomarkers in response to determine that one or more required ultrasound biomarkers are missing from the received or extracted set of ultrasound biomarkers. The request can comprise an identification of the missing one or more required ultrasound biomarkers, instructions for obtaining the missing one or more required ultrasound biomarkers, and/or any other information. The request can be provided to another system, or to a medical professional such as through a user interface. The request can be communicated locally or remotely.

At optional step 136 of the method, the system receives at least one of the one or more missing ultrasound biomarkers in response to communicating the request. For example, a medical professional may receive the request and then may obtain the one or more missing ultrasound biomarkers, such as by performing additional analysis of ultrasound imaging, or by performing additional ultrasound imaging to obtain the missing information. According to another embodiment, the request is communicated to another system such as an ultrasound device or analysis system which can automatically extract or otherwise identify the one or more missing ultrasound biomarkers, and which can automatically provide the obtained data back to the patient analysis system.

At step 122 of the method, the patient analysis system receives or obtains a cardiac stiffness measurement for the subject's heart. The cardiac stiffness measurement for the subject's heart can be obtained using one or more of a plurality of different methods for obtaining such a measurement.

According to an embodiment, left ventricle (LV) filling following atrial kick (AK) in late diastole generates LV myocardial stretch that propagates with a speed related to myocardial stiffness. Changes in myocardial stiffness have shown to be related to cardiac disease, particularly HFpEF, and therefore a cardiac stiffness measurement tool supplements differential diagnosis of HFpEF. However, cardiac stiffness measurement is not part of the existing workflow in hospitals, and thus using cardiac stiffness as an input brings added value and improved accuracy and reproducibility in estimating the likelihood of the HFpEF etiologies.

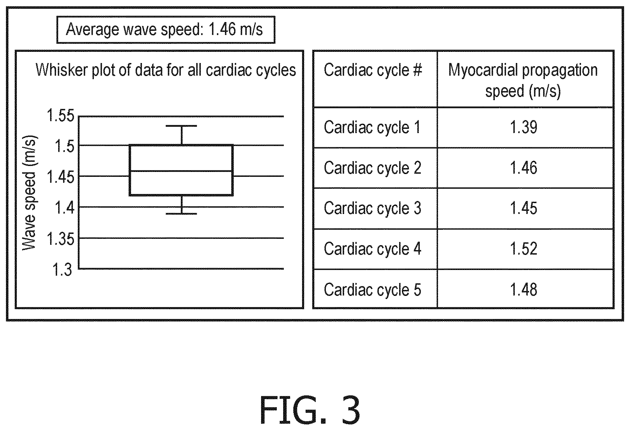

According to an embodiment, the cardiac stiffness measurement for the subject's heart can be obtained using a semi-automatic methodology for a noninvasive estimate of cardiac stiffness where a combination of high frame imaging modes and an algorithm capable of processing the tissue imaging automatically computes the heart tissue elasticity. Referring to FIG. 3, in one embodiment, is a graphic depicting an output of the proposed cardiac stiffness feature, including the average cardiac stiffness (wave speed value), all valid speed measurements, and a whisker plot showing the variability.

According to an embodiment, the cardiac stiffness (wave speed) measurements can be based on other methods. For example, some HFpEF patients may experience symptoms of atrial fibrillation and since the AK is lost in those patients, measurement of cardiac stiffness using an AK-based feature may not be optimal. **In** those group of HFpEF patients, the cardiac stiffness can be measured based on other methods such as natural shear wave speed measurement after mitral valve closure (during late diastole) and aortic valve closure (early diastole) or external shear wave speed measurements using push pulses produced by the ultrasound probe itself. The results of the stiffness measurements can then be utilized by downstream steps of the method for the estimation of HFpEF etiologies likelihood.

The cardiac stiffness measurement received by or extracted by the patient analysis system can be utilized, before or after data processing, immediately or may be stored in local or remote storage for use in further steps of the method.

At step 124 of the method, the patient analysis system receives clinical information about the subject. The clinical information about the subject can be any information relevant to or useful in any downstream step of the method, including as input to the trained heart failure model configured to output a likelihood of at least one of a plurality of HFpEF etiologies. According to an embodiment, the clinical information about the subject comprises one or more of an ultrasound exam type, a reason for the ultrasound analysis, age of the subject, sex of the subject, body mass index of the subject, atrial fibrillation status or diagnosis, coronary artery disease status or diagnosis, medical treatment, and medical diagnosis, among many other types of clinical information. For example, age may affect the interpretation of diastolic parameters including E (mitral inflow velocity at early diastole), A (mitral inflow velocity at late diastole), and E/A. Additionally, the evaluation of A and E/A is challenging in AF patients and E is challenging in patients with a history of CAD. Therefore, these clinical information data can be significant factors affecting HFpEF diagnosis. Accordingly, the received information can be any information relevant to a patient analysis as described or otherwise envisioned herein.

The patient analysis system can receive patient clinical information from a variety of different sources. According to an embodiment, the patient analysis system is in communication with an electronic medical records database from which the patient clinical information may be obtained or received. According to an embodiment, the patient analysis system comprises an electronic medical record database or system 270 which is optionally in direct and/or indirect communication with system 200. According to another embodiment, the patient analysis system may obtain or receive the information from equipment or a healthcare professional obtaining that information directly from the patient.

The patient clinical information received by the patient analysis system may be processed by the system according to methods for data handling and processing/preparation, including but not limited to the methods described or otherwise envisioned herein. The patient clinical information received by the patient analysis system may be utilized, before or after processing, immediately or may be stored in local or remote storage for use in further steps of the method.

At optional step 126 of the method, the patient analysis system receives the results of one or more previous imaging analyses of the subject's heart. According to an embodiment, the imaging analysis is ultrasound imaging or another imaging modality. According to an embodiment, a previous cardiac MR image or CT image may affect the final diagnostic decision, especially if there are discrepancies between the parameters obtained from the ultrasound and parameters obtained from an MRI, such as different longitudinal strain value in ultrasound and MR.

At optional step 128 of the method, the patient analysis system receives one or more ultrasound biomarkers for the subject from a previous imaging analysis obtained for the subject. According to an embodiment, ultrasound biomarkers from previous analyses can provide additional diagnostic values in terms of trending data. For example, if the suspicion of amyloidosis biomarkers goes up from the previous exam to the current exam, that could be a red flag for amyloidosis. Many other examples are possible.

The received results of one or more previous imaging analyses of the subject's heart, and/or received one or more ultrasound biomarkers for the subject from a previous imaging analysis obtained for the subject may be utilized, before or after processing, immediately or may be stored in local or remote storage for use in further steps of the method.

At step 140 of the method, a trained heart failure model of the patient analysis system analyzes the received input to generate a likelihood of at least one of a plurality of HFpEF etiologies. The generated likelihood of at least one of a plurality of HFpEF etiologies comprises a likelihood that the subject is experiencing heart failure with preserved ejection fraction. According to an embodiment, the plurality of HFpEF etiologies comprises cardiac amyloidosis, coronary artery disease, hypertension, pericardial disease, hypertrophic cardiomyopathy, and valvular disease, although fewer or more HFpEF etiologies are possible.

According to an embodiment, the input to the trained heart failure model of the patient analysis system comprises the extracted plurality of ultrasound biomarkers, the received cardiac stiffness measurement, and the received clinical information about the subject. According to another embodiment, the input to the trained heart failure model of the patient analysis system further comprises the received results of one or more previous imaging analyses of the subject's heart, and/or the received one or more ultrasound biomarkers for the subject from a previous imaging analysis obtained for the subject. Other input to the trained heart failure model is possible.

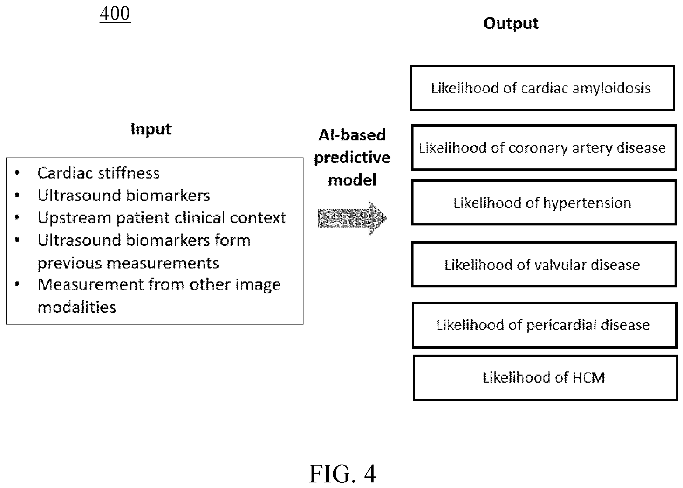

Referring to Fig. 4, in one embodiment, is a graphic 400 depicting input to and output from the trained heart failure model of the patient analysis system. According to this embodiment, the input to the trained heart failure model of the patient analysis system comprises one or more of the received cardiac stiffness measurement, the extracted plurality of ultrasound biomarkers, the received clinical information about the subject, the received one or more ultrasound biomarkers for the subject from a previous imaging analysis obtained for the subject, and/or the received results of one or more previous imaging analyses of the subject's heart. According to this embodiment, the output from the trained heart failure model of the patient analysis system comprises a likelihood for one or more HFpEF etiologies such as cardiac amyloidosis, coronary artery disease, hypertension, valvular disease pericardial disease, and hypertrophic cardiomyopathy, although fewer or more HFpEF etiologies are possible.

According to an embodiment, the trained heart failure model of the patient analysis system can generate a likelihood of at least one of a plurality of HFpEF etiologies using a wide variety of different classifier and/or machine learning algorithms as described or otherwise envisioned herein. According to an embodiment, the trained heart failure model of the patient analysis system can be trained according to a wide variety of methods and approaches. As one example, the model may comprise a neural network approach.

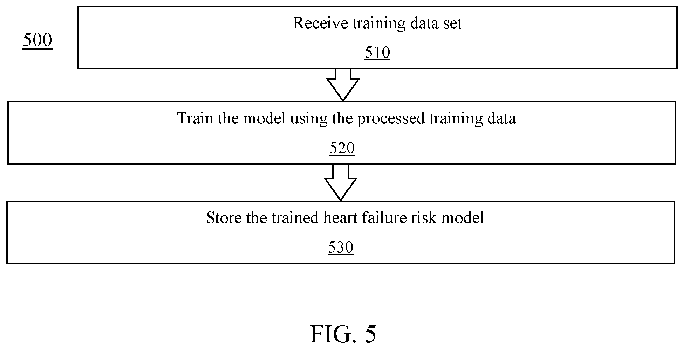

Referring to Fig. 5, in one embodiment, is a flowchart of a method 500 for training the heart failure model of the patient analysis system. At step 510 of the method, the system receives a training data set comprising training data about a plurality of patients, such as historical patient data. The training data can comprise input such as one or more of cardiac stiffness measurements, ultrasound biomarkers, clinical information about the patients, received one or more ultrasound biomarkers for the patients from a previous imaging analysis obtained for the patients, and/or the received results of one or more previous imaging analyses of the patients' heart. The training data can also comprise a diagnosis of HFpEF or no HFpEF for each of the plurality of patients. The training data may be stored in and/or received from one or more databases. The database may be a local and/or remote database. For example, the patient readmission risk analysis system may comprise a database of training data.

According to an embodiment, the patient analysis system may comprise a data pre-processor or similar component or algorithm configured to process the received training data. For example, the data pre-processor analyzes the training data to remove noise, bias, errors, and other potential issues. The data pre-processor may also analyze the input data to remove low-quality data. Many other forms of data pre-processing or data point identification and/or extraction are possible.

At step 520 of the method, the system trains the machine learning algorithm, which will be the algorithm utilized in analyzing the input information as described or otherwise envisioned. The machine learning algorithm is trained using the training data set according to known methods for training a machine learning algorithm. According to an embodiment, the algorithm is trained, using the processed training dataset, to generate a likelihood of at least one of a plurality of HFpEF etiologies. The generated likelihood of at least one of a plurality of HFpEF etiologies comprises a likelihood that the subject is experiencing heart failure with preserved ejection fraction. According to an embodiment, the algorithm is also trained, using the processed training dataset, to generate one or more intervention recommendations based on the determined one or more likelihoods.

At step 530 of the method, the trained heart failure model of the patient analysis system is stored for future use. According to an embodiment, the model may be stored in local or remote storage.

According to an embodiment, ground truth for the expected likelihood of different HFpEF etiologies can be collected from the definite results of the invasive/minimally invasive follow-up diagnostic tests (biopsy, PET, or CMR) obtained from HFpEF patients in a retrospective or prospective study, which are analyzed by an expert panel of cardiologists for each given upstream patient's clinical context. The combined data (events in the current exam obtained from ultrasound imaging and cardiac stiffness measurement, previous measurements of other image modalities, and historical clinical parameters obtained from electronic medical records) can then be stored and processed for learning or display using a proposed AI model. This supervised learning approach could be an institution-agnostic tool for differential HFpEF likelihood estimation. The accuracy of an AI-based learning network can get stronger over time by adding more data to it, such as a self-learning algorithm.

Returning to method 100 in Fig. 1, at step 150 of the method a visualization of the generated likelihood of at least one of a plurality of HFpEF etiologies is displayed to a medical professional or other user via a user interface of the patient analysis system. According to an embodiment, the plurality of HFpEF etiologies comprises cardiac amyloidosis, coronary artery disease, hypertension, pericardial disease, hypertrophic cardiomyopathy, and valvular disease. According to an embodiment, the display may further comprise one or more of the subject's name, one or more details about the ultrasound analysis, a likelihood of each of each of a plurality of HFpEF etiologies, and/or a treatment recommendation, among many other types of information.

According to an embodiment, the information may be communicated by wired and/or wireless communication to a user interface and/or to another device. For example, the system may communicate the information to a mobile phone, computer, laptop, wearable device, and/or any other device configured to allow display and/or other communication of the report. The user interface can be any device or system that allows information to be conveyed and/or received, and may include a display, a mouse, and/or a keyboard for receiving user commands.

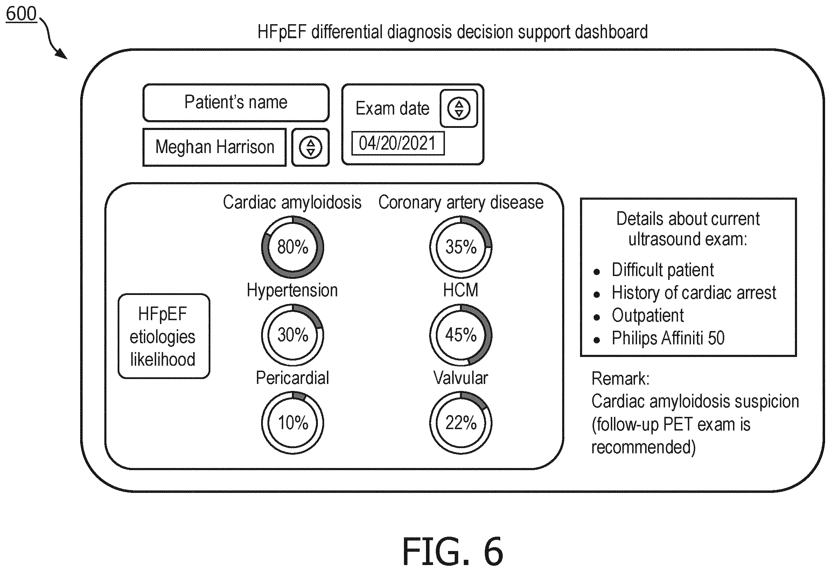

Referring to Fig. 6, in one embodiment, is a schematic representation of a possible visualization of the generated likelihood of a a plurality of HFpEF etiologies, including cardiac amyloidosis, coronary artery disease, hypertension, pericardial disease, hypertrophic cardiomyopathy, and valvular disease. For example, the likelihood of the cardiac amyloidosis HFpEF etiology is 80%, which may be above a predetermined threshold for concern, alarm, or other threshold. The 80% likelihood results in the provided remark "Cardiac Amyloidosis suspicion" and a recommendation "Follow-up PET exam is recommended." The display also comprises information including the patient's name, the exam date, and details about the current ultrasound exam.

According to an embodiment of a patient analysis system, the system can comprise a user interface to facilitate the methods described or otherwise envisioned herein. Accordingly, the user interface can comprise a "decision support tool for differential HFpEF diagnosis" button or activator that appears on the ultrasound scanner touch panel or a workspace such as a Philips Intellispace Cardiovascular (ISCV) platform for the user to launch the application.

According to an embodiment of a patient analysis system, the system may ask the user to activate an automatic cardiac stiffness measurement tool (for the current acquisition on the scanner) or load the previous stiffness measurement results (in case the results are already available).

According to an embodiment of a patient analysis system, the AI predictive model of the patient analysis system is automatically implemented using the inputs including the stiffness, other ultrasound biomarkers, previous measurements from other modalities, and/or upstream patient clinical context. If some of the ultrasound biomarkers are missing, the user is asked to provide/measure them, including using automated measurement tools.

According to an embodiment of a patient analysis system, the system includes a user interface dashboard that shows the likelihood of different HFpEF etiologies. Some remarks and recommendation may also appear in the user interface to recommend the next steps to the user.

In addition to the HFpEF etiologies likelihood, the other available in-house ultrasound-based features related to the cardiac diagnostic domain (such as the LA index tool, reconstructed PV loops, calcification score, etc.) can be provided in the dashboard for additional clinical decision support for the HFpEF differential diagnosis.

At optional step 160 of method 100 depicted in Fig. 1, the visualization of the generated likelihood can be utilized by a healthcare professional to implement a healthcare treatment for the subject. For example, a clinician or other decisionmaker utilizes the displayed generated likelihood of one or more HFpEF etiologies for patient care decision-making. For example, the healthcare recommendation may comprise a recommendation to initiate, continue, or stop a particular treatment configured to address one or more HFpEF etiologies, which is based on the determined likelihood of the one or more HFpEF etiologies. Implementation can comprise a prescription, order, additional testing, and/or other implementation. Many other implementations are possible.

Referring to Fig. 2 is a schematic representation of a patient analysis system 200. System 200 may be any of the systems described or otherwise envisioned herein, and may comprise any of the components described or otherwise envisioned herein. It will be understood that FIG. 2 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. **In** various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. **In** this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, the electronic medical record system 270 is an electronic medical records database from which the information about the patient, including the clinical information, may be obtained or received. The electronic medical records database may be a local or remote database and is in direct and/or indirect communication with the patient analysis system 200. Thus, according to an embodiment, the patient analysis system comprises an electronic medical record database or system 270.

According to an embodiment, the system comprises one or more ultrasound devices 280 capable of acquiring the required ultrasound images or analysis. According to another embodiment, patient analysis system 200 is in wired and/or wireless communication with a local or remote ultrasound device 280 capable of acquiring the required ultrasound images or analysis. According to another embodiment, patient analysis system 200 is in wired and/or wireless communication with a local or remote database 280 which stores the ultrasound images or analysis. The patient analysis system 200 can obtain the required ultrasound images or analysis from one or more of these sources.

According to an embodiment, storage 260 of system 200 may store one or more algorithms, modules, and/or instructions to carry out one or more functions or steps of the methods described or otherwise envisioned herein. For example, the system may comprise, among other instructions or data, ultrasound biomarker extraction instructions 262, a trained heart failure model 263, and/or reporting instructions 264.

According to an embodiment, ultrasound biomarker extraction instructions 262 direct the system to extract a plurality of ultrasound biomarkers from received result of an ultrasound analysis. The plurality of ultrasound biomarkers can be any metrics, measurements, parameters, or other data extracted from an ultrasound analysis, including but not limited to one or more of ejection fraction, global longitudinal strain, flow propagation velocity, mitral inflow velocity at early diastole, mitral inflow velocity at late diastole, mitral annular velocity at early diastole, mitral annular velocity at late diastole, left atrium volume index, left ventricle thickness, septum thickness, thickness of one or more valves, right ventricle thickness, relative wall thickness, tricuspid regurgitation velocity, and left ventricle mass index. The ultrasound biomarkers can be extracted using any of a wide variety of methods for data extraction from ultrasound imaging. According to an embodiment, one or more of the plurality of ultrasound biomarkers are obtained or extracted automatically by software or algorithm associated with the ultrasound device or the patient analysis system. For example, one or more of the plurality of ultrasound biomarkers can be obtained with an ultrasound workstation solution. According to another embodiment, one or more of the plurality of ultrasound biomarkers are obtained or extracted manually during the ultrasound analysis.

According to an embodiment, the trained heart failure model 263 is configured to generate a likelihood of at least one of a plurality of HFpEF etiologies. The generated likelihood of at least one of a plurality of HFpEF etiologies comprises a likelihood that the subject is experiencing heart failure with preserved ejection fraction. According to an embodiment, the plurality of HFpEF etiologies comprises cardiac amyloidosis, coronary artery disease, hypertension, pericardial disease, hypertrophic cardiomyopathy, and valvular disease, although fewer or more HFpEF etiologies are possible. According to an embodiment, the input to the trained heart failure model of the patient analysis system comprises the extracted plurality of ultrasound biomarkers, the received cardiac stiffness measurement, and the received clinical information about the subject. According to another embodiment, the input to the trained heart failure model of the patient analysis system further comprises the received results of one or more previous imaging analyses of the subject's heart, and/or the received one or more ultrasound biomarkers for the subject from a previous imaging analysis obtained for the subject. Other input to the trained heart failure model is possible. The trained heart failure model 263 is trained using a training data set as described or otherwise envisioned herein.

According to an embodiment, reporting instructions 264 direct the system to generate and provide to a user via a user interface information comprising a generated visualization of the generated likelihood of at least one of a plurality of HFpEF etiologies. According to an embodiment, the display may further comprise one or more of the subject's name, one or more details about the ultrasound analysis, a likelihood of each of each of a plurality of HFpEF etiologies, and/or a treatment recommendation, among many other types of information. Any of the information may be communicated by wired and/or wireless communication via the user interface of the system or of another device. For example, the system may communicate the information to a mobile phone, computer, laptop, wearable device, and/or any other device configured to allow display and/or other communication of the report. The user interface can be any device or system that allows information to be conveyed and/or received, and may include a display, a mouse, and/or a keyboard for receiving user commands.

Accordingly, within the context of the disclosure herein, aspects of the embodiments may take the form of a computer program product embodied in one or more non-transitory computer readable media having computer readable program code embodied thereon. Thus, according to one embodiment is a non-transitory computer-readable storage medium comprising computer program code instructions which, when executed by a processor, enables the processor to carry out a method including: (i) receiving a result of an ultrasound analysis of the subject's heart from a current ultrasound exam; (ii) extracting, from the received result of the ultrasound analysis, a plurality of ultrasound biomarkers; (iii) receiving a cardiac stiffness measurement for the subject's heart from the current ultrasound exam and/or from a previous ultrasound exam; (iv) receiving clinical information about the subject; (v) analyzing, using a trained heart failure model, the extracted plurality of ultrasound biomarkers, the received cardiac stiffness measurement, and the received clinical information about the subject to generate a likelihood of at least one of a plurality of HFpEF etiologies, wherein the generated likelihood of at least one of a plurality of HFpEF etiologies comprises a likelihood that the subject is experiencing heart failure with preserved ejection fraction; and (vi) displaying, on a user interface, a visualization of the generated likelihood of at least one of a plurality of HFpEF etiologies. The program code may perform entirely on a user's computer, partly on a user's computer, as a stand-alone software package, partly on a user's computer and partly on a remote computer, or entirely on a remote computer or server.

According to an embodiment, the patient analysis system is configured to process many thousands or millions of datapoints in the input data used to train the system, as well as to process and analyze the received plurality of mobility features. For example, generating a functional and skilled trained system using an automated process such as feature identification and extraction and subsequent training requires processing of millions of datapoints from input data and the generated features. This can require millions or billions of calculations to generate a novel trained system from those millions of datapoints and millions or billions of calculations. As a result, the trained system is novel and distinct based on the input data and parameters of the machine learning algorithm, and thus improves the functioning of the patient analysis system. Thus, generating a functional and skilled trained system comprises a process with a volume of calculation and analysis that a human brain cannot accomplish in a lifetime, or multiple lifetimes. By providing an improved patient analysis, this novel patient analysis system has an enormous positive effect on patient diagnosis and care compared to prior art systems.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of.".

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. It is, therefore, to be understood that the foregoing embodiments are presented by way of example only and that, within the scope of the appended claims, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A computer implemented method (100) for providing a visualized likelihood of heart failure with preserved ejection fraction, HFpEF, for a subject, comprising:
receiving (120) a result of an ultrasound analysis of the subject's heart from a current ultrasound exam;
extracting (130), from the received result of the ultrasound analysis, a plurality of ultrasound biomarkers;
receiving (122) a cardiac stiffness measurement for the subject's heart from the current exam and/or from a previous ultrasound exam;
receiving (124) clinical information about the subject;
analyzing (140), using a trained heart failure model, the extracted plurality of ultrasound biomarkers, the received cardiac stiffness measurement, and the received clinical information about the subject to generate a likelihood of at least one of a plurality of HFpEF etiologies, wherein the generated likelihood of at least one of a plurality of HFpEF etiologies comprises a likelihood that the subject is experiencing heart failure with preserved ejection fraction;
displaying (150), on a user interface, a visualization of the generated likelihood of at least one of a plurality of HFpEF etiologies.

2. The method of claim 1, wherein the plurality of HFpEF etiologies comprises cardiac amyloidosis, coronary artery disease, hypertension, pericardial disease, hypertrophic cardiomyopathy, and valvular disease.

3. The method of claim 1, further comprising the steps:
receiving (126) results of one or more previous imaging analyses of the subject's heart, wherein the imaging analysis is ultrasound imaging or another imaging modality; and
receiving (128) one or more ultrasound biomarkers from a previous imaging analysis;
wherein analyzing (140), using a trained heart failure model, further comprises the received one or more previous imaging analyses and/or the one or more ultrasound biomarkers from a previous imaging analysis.

4. The method of claim 1, wherein displaying further comprises displaying, on the user interface, the following: (i) the subject's name; (ii) one or more details about the ultrasound analysis; (iii) a likelihood of each of each of a plurality of HFpEF etiologies; and (iv) a treatment recommendation.

5. The method of claim 1, further comprising the steps of:
determining (132) that one or more ultrasound biomarkers are missing from the extracted plurality of ultrasound biomarkers;
generating (134) a request for the one or more missing ultrasound biomarkers; and
receiving (136), in response to the request, at least one of the one or more missing ultrasound biomarkers.

6. The method of claim 1, wherein the plurality of ultrasound biomarkers comprises one or more of ejection fraction, global longitudinal strain, flow propagation velocity, mitral inflow velocity at early diastole, mitral inflow velocity at late diastole, mitral annular velocity at early diastole, mitral annular velocity at late diastole, left atrium volume index, left ventricle thickness, septum thickness, thickness of one or more valves, right ventricle thickness, relative wall thickness, tricuspid regurgitation velocity, and left ventricle mass index.

7. The method of claim 1, wherein the clinical information about the subject comprises one or more of an ultrasound exam type, a reason for the ultrasound analysis, age of the subject, sex of the subject, body mass index of the subject, atrial fibrillation status or diagnosis, and coronary artery disease status or diagnosis.

8. The method of claim 1, wherein cardiac stiffness is measured by an atrial kick method, a valve closure method, and/or an external shear wave method.

9. A system (200) for providing a visualized likelihood of heart failure with preserved ejection fraction, HFpEF, for a subject, comprising:
a trained heart failure model (263);
a processor (220) configured to: (i) receive a result of an ultrasound analysis of the subject's heart from a current ultrasound exam; (ii) extract, from the received result of the ultrasound analysis, a plurality of ultrasound biomarkers; (iii) receive a cardiac stiffness measurement for the subject's heart from the current exam and/or from a previous ultrasound exam; (iv) receive clinical information about the subject; (v) analyze, using the trained heart failure model, the extracted plurality of ultrasound biomarkers, the received cardiac stiffness measurement, and the received clinical information about the subject to generate a likelihood of at least one of a plurality of HFpEF etiologies, wherein the generated likelihood of at least one of a plurality of HFpEF etiologies comprises a likelihood that the subject is experiencing heart failure with preserved ejection fraction; and (vi) generate a visualization of the generated likelihood of at least one of a plurality of HFpEF etiologies; and
a user interface (240) configured to provide the generated likelihood of at least one of a plurality of HFpEF etiologies.

10. The system of claim 9, wherein the plurality of HFpEF etiologies comprises cardiac amyloidosis, coronary artery disease, hypertension, pericardial disease, hypertrophic cardiomyopathy, and valvular disease.

11. The system of claim 9, wherein the user interface is further configured to display: (i) the subject's name; (ii) one or more details about the ultrasound analysis; (iii) a likelihood of each of each of a plurality of HFpEF etiologies; and (iv) a treatment recommendation.

12. The system of claim 9, wherein the processor is further configured to: determine that one or more ultrasound biomarkers are missing from the extracted plurality of ultrasound biomarkers; (ii) generate a request for the one or more missing ultrasound biomarkers; and receive, in response to the request, at least one of the one or more missing ultrasound biomarkers.

13. The system of claim 9, wherein the plurality of ultrasound biomarkers comprises one or more of ejection fraction, global longitudinal strain, flow propagation velocity, mitral inflow velocity at early diastole, mitral inflow velocity at late diastole, mitral annular velocity at early diastole, mitral annular velocity at late diastole, left atrium volume index, left ventricle thickness, septum thickness, thickness of one or more valves, right ventricle thickness, relative wall thickness, tricuspid regurgitation velocity, and left ventricle mass index.

14. The system of claim 9, wherein: (i) the clinical information about the subject comprises one or more of an ultrasound exam type, a reason for the ultrasound analysis, age of the subject, sex of the subject, body mass index of the subject, atrial fibrillation status or diagnosis, and coronary artery disease status or diagnosis, and/or (ii) cardiac stiffness is measured by an atrial kick method, a valve closure method, and/or an external shear wave method.

15. A non-transitory computer-readable storage medium comprising computer program code instructions which, when executed by a processor, enables the processor to carry out the method according to claim 1.

## Patentansprüche

1. Computerimplementiertes Verfahren (100) zum Bereitstellen einer visualisierten Wahrscheinlichkeit einer Herzinsuffizienz mit erhaltener Ejektionsfraktion, HFpEF, für ein Subjekt, umfassend:
Empfangen (120) eines Ergebnisses einer Ultraschallanalyse des Herzens des Subjekts aus einer aktuellen Ultraschalluntersuchung;
Extrahieren (130) aus dem empfangenen Ergebnis der Ultraschallanalyse einer Vielzahl von Ultraschall-Biomarkern;
Empfangen (122) einer Herzsteifigkeitsmessung für das Herz des Subjekts aus der aktuellen Untersuchung und/oder von einer vorherigen Ultraschalluntersuchung;
Empfangen (124) klinischer Informationen über das Subjekt;
Analysieren (140) unter Verwendung eines trainierten Herzinsuffizienzmodells der extrahierten Vielzahl von Ultraschall-Biomarkern, der empfangenen Herzsteifigkeitsmessung und der empfangenen klinischen Informationen über das Subjekt, um eine Wahrscheinlichkeit für mindestens eine einer Vielzahl von HFpEF-Ätiologien zu erzeugen, wobei die erzeugte Wahrscheinlichkeit für mindestens eine der Vielzahl von HFpEF-Ätiologien eine Wahrscheinlichkeit umfasst, dass das Subjekt an Herzinsuffizienz mit erhaltener Ejektionsfraktion leidet;
Anzeigen (150) auf einer Benutzeroberfläche einer Visualisierung der erzeugten Wahrscheinlichkeit von mindestens einer von einer Vielzahl von HFpEF-Ätiologien.

2. Verfahren nach Anspruch 1, wobei die Vielzahl von HFpEF-Ätiologien kardiale Amyloidose, koronare Herzkrankheit, Hypertonie, Perikarderkrankung, hypertrophe Kardiomyopathie und Herzklappenerkrankung umfasst.

3. Verfahren nach Anspruch 1, weiter die Schritte umfassend:
Empfangen (126) von Ergebnissen einer oder mehrerer vorheriger Bildgebungsanalysen des Herzens des Subjekts, wobei die Bildgebungsanalyse eine Ultraschallbildgebung oder eine andere Bildgebungsmodalität ist; und
Empfangen (128) eines oder mehrerer Ultraschall-Biomarker aus einer vorherigen Bildgebungsanalyse;
wobei Analysieren (140) unter Verwendung eines trainierten Herzinsuffizienzmodells weiter die empfangene(n) eine oder mehreren vorherige(n) Bildgebungsanalyse(n) und/oder den einen oder mehrere Ultraschall-Biomarker aus einer vorherigen Bildgebungsanalyse umfasst.

4. Verfahren nach Anspruch 1, wobei Anzeigen weiter Anzeigen auf der Benutzeroberfläche des Folgenden umfasst: (i) des Namens des Subjekts; (ii) eines oder mehrerer Details zur Ultraschallanalyse; (iii) einer Wahrscheinlichkeit für jede einzelne einer Vielzahl von HFpEF-Ätiologien; und (iv) einer Behandlungsempfehlung.

5. Verfahren nach Anspruch 1, weiter umfassend die Schritte zum:
Bestimmen (132), dass ein oder mehrere Ultraschall-Biomarker in der extrahierten Vielzahl von Ultraschall-Biomarkern fehlen;
Erzeugen (134) einer Anforderung für den einen oder mehrere fehlende Ultraschall-Biomarker; und
Empfangen (136), als Reaktion auf die Anforderung, mindestens eines des einen oder der mehreren fehlenden Ultraschall-Biomarker.

6. Verfahren nach Anspruch 1, wobei die Vielzahl von Ultraschall-Biomarkern eines oder mehrere von Ejektionsfraktion, globale Längsspannung, Flussausbreitungsgeschwindigkeit, Mitralklappeneinflussgeschwindigkeit in früher Diastole, Mitralklappeneinflussgeschwindigkeit in später Diastole, Mitralklappenringgeschwindigkeit in früher Diastole, Mitralklappenringgeschwindigkeit in später Diastole, linker Vorhofvolumenindex, Dicke des linken Ventrikels, Septumdicke, Dicke einer oder mehrerer Klappen, Dicke des rechten Ventrikels, relative Wanddicke, Trikuspidalklappeninsuffizienzgeschwindigkeit und linker Ventrikelmassenindex umfasst.

7. Verfahren nach Anspruch 1, wobei die klinischen Informationen über das Subjekt eines oder mehrere von einer Art der Ultraschalluntersuchung, eines Grunds für die Ultraschallanalyse, Alters des Subjekts, Geschlechts des Subjekts, Body-Mass-Index des Subjekts, Vorhofflimmern-Status oder -Diagnose und Status oder Diagnose einer koronaren Herzkrankheit umfasst.

8. Verfahren nach Anspruch 1, wobei die Herzsteifigkeit durch ein Vorhofkickverfahren, ein Klappenverschlussverfahren und/oder ein externes Scherwellenverfahren gemessen wird.

9. System (200) zum Bereitstellen einer visualisierten Wahrscheinlichkeit einer Herzinsuffizienz mit erhaltener Ejektionsfraktion, HFpEF, für ein Subjekt, umfassend:
ein trainiertes Herzinsuffizienzmodell (263);
einen Prozessor (220), der konfiguriert ist, um: (i) ein Ergebnis einer Ultraschallanalyse des Herzens des Subjekts aus einer aktuellen Ultraschalluntersuchung zu empfangen; (ii) aus dem empfangenen Ergebnis der Ultraschallanalyse eine Vielzahl von Ultraschall-Biomarkern zu extrahieren; (iii) eine Messung der Herzsteifigkeit für das Herz des Subjekts aus der aktuellen Untersuchung und/oder aus einer vorherigen Ultraschalluntersuchung zu empfangen; (iv) klinische Informationen über das Subjekt zu empfangen; (v) unter Verwendung des trainierten Herzinsuffizienzmodells die extrahierte Vielzahl von Ultraschall-Biomarkern, die empfangene Herzsteifigkeitsmessung und die empfangenen klinischen Informationen über das Subjekt zu analysieren, um eine Wahrscheinlichkeit für mindestens eine von mehreren HFpEF-Ätiologien zu erzeugen, wobei die erzeugte Wahrscheinlichkeit für mindestens eine von mehreren HFpEF-Ätiologien eine Wahrscheinlichkeit umfasst, dass das Subjekt an Herzinsuffizienz mit erhaltener Ejektionsfraktion leidet; und (vi) eine Visualisierung der erzeugten Wahrscheinlichkeit für mindestens eine von mehreren HFpEF-Ätiologien zu erzeugen; und
eine Benutzeroberfläche (240), die konfiguriert ist, um die erzeugte Wahrscheinlichkeit mindestens einer von einer Vielzahl von HFpEF-Ätiologien bereitzustellen.

10. System nach Anspruch 9, wobei die Vielzahl von HFpEF-Ätiologien kardiale Amyloidose, koronare Herzkrankheit, Hypertonie, Perikarderkrankung, hypertrophe Kardiomyopathie und Herzklappenerkrankung umfasst.

11. System nach Anspruch 9, wobei die Benutzeroberfläche weiter konfiguriert ist, um anzuzeigen: (i) den Namen des Subjekts; (ii) ein oder mehrere Details zur Ultraschallanalyse; (iii) eine Wahrscheinlichkeit für jede einzelne einer Vielzahl von HFpEF-Ätiologien; und (iv) eine Behandlungsempfehlung.

12. System nach Anspruch 9, wobei der Prozessor weiter konfiguriert ist, um zu bestimmen, dass ein oder mehrere Ultraschall-Biomarker in der extrahierten Vielzahl von Ultraschall-Biomarkern fehlen; und (ii) eine Anforderung für den einen oder die mehreren fehlenden Ultraschall-Biomarker zu erzeugen; und als Reaktion auf die Anforderung mindestens einen des einen oder mehrerer fehlender Ultraschall-Biomarker zu empfangen.

13. System nach Anspruch 9, wobei die Vielzahl von Ultraschall-Biomarkern eines oder mehrere von Ejektionsfraktion, globale Längsspannung, Flussausbreitungsgeschwindigkeit, Mitralklappeneinflussgeschwindigkeit in früher Diastole, Mitralklappeneinflussgeschwindigkeit in später Diastole, Mitralklappenringgeschwindigkeit in früher Diastole, Mitralklappenringgeschwindigkeit in später Diastole, linker Vorhofvolumenindex, Dicke des linken Ventrikels, Septumdicke, Dicke einer oder mehrerer Klappen, Dicke des rechten Ventrikels, relative Wanddicke, Trikuspidalklappeninsuffizienzgeschwindigkeit und linker Ventrikelmassenindex umfasst.

14. System nach Anspruch 9, wobei: (i) die klinischen Informationen über das Subjekt eines oder mehrere von einer Art der Ultraschalluntersuchung, eines Grunds für die Ultraschallanalyse, Alters des Subjekts, Geschlechts des Subjekts, Body-Mass-Index des Subjekts, Vorhofflimmern-Status oder -Diagnose und Status oder Diagnose einer koronaren Herzkrankheit umfasst, und/oder (ii) die Herzsteifigkeit mit einem Vorhofkickverfahren und einem Klappenverschlussverfahren und/oder einem externen Scherwellenverfahren gemessen wird.

15. Nicht flüchtiges computerlesbares Speichermedium, das Computerprogrammcode-Anweisungen umfasst, die, wenn sie von einem Prozessor ausgeführt werden, den Prozessor in die Lage versetzen, das Verfahren nach Anspruch 1 durchzuführen.

## Revendications

1. Procédé mis en œuvre par ordinateur (100) pour fournir une probabilité visualisée d'une insuffisance cardiaque avec une fraction d'éjection préservée (HFpEF) pour un sujet, comprenant :
la réception (120) d'un résultat d'une analyse par ultrasons du cœur du sujet à partir d'un examen par ultrasons actuel ;
l'extraction (130), à partir du résultat reçu de l'analyse par ultrasons, d'une pluralité de biomarqueurs ultrasonores ;
la réception (122) d'une mesure de rigidité cardiaque du cœur du sujet à partir de l'examen actuel et/ou d'un précédent examen par ultrasons ;
la réception (124) d'informations cliniques concernant le sujet ;
l'analyse (140), à l'aide d'un modèle d'insuffisance cardiaque entraîné, de la pluralité extraite de biomarqueurs ultrasonores, de la mesure de rigidité cardiaque reçue et des informations cliniques reçues concernant le sujet pour générer une probabilité d'au moins une étiologie HFpEF d'une pluralité d'étiologies HFpEF, dans lequel la probabilité générée d'au moins une étiologie HFpEF d'une pluralité d'étiologies HFpEF comprend une probabilité que le sujet souffre d'une insuffisance cardiaque avec une fraction d'éjection préservée ;
l'affichage (150), sur une interface utilisateur, d'une visualisation de la probabilité générée d'au moins une étiologie HFpEF d'une pluralité d'étiologies HFpEF.

2. Procédé selon la revendication 1, dans lequel la pluralité d'étiologies HFpEF comprend une amylose cardiaque, une maladie coronarienne, l'hypertension, une maladie péricardique, la cardiomyopathie hypertrophique et une maladie valvulaire.

3. Procédé selon la revendication 1, comprenant en outre les étapes consistant à :
recevoir (126) des résultats d'une ou de plusieurs précédentes analyses d'imagerie du cœur du sujet, dans lequel l'analyse d'imagerie est une imagerie par ultrasons ou une autre modalité d'imagerie ; et
recevoir (128) un ou plusieurs biomarqueurs ultrasonores provenant d'une précédente analyse d'imagerie ;
dans lequel l'analyse (140), à l'aide d'un modèle d'insuffisance cardiaque entraîné, comprend en outre les une ou plusieurs précédentes analyses d'imagerie reçues et/ou les un ou plusieurs biomarqueurs ultrasonores provenant d'une précédente analyse d'imagerie.

4. Procédé selon la revendication 1, dans lequel l'affichage comprend en outre l'affichage, sur l'interface utilisateur, des éléments suivants : (i) le nom du sujet ; (ii) un ou plusieurs détails concernant l'analyse par ultrasons ; (iii) une probabilité de chaque étiologie HFpEF d'une pluralité d'étiologies HFpEF ; et (iv) une recommandation de traitement.

5. Procédé selon la revendication 1, comprenant en outre les étapes consistant à :
déterminer (132) qu'un ou plusieurs biomarqueurs ultrasonores sont manquants dans la pluralité extraite de biomarqueurs ultrasonores ;
générer (134) une demande pour les un ou plusieurs biomarqueurs ultrasonores manquants ; et
recevoir (136), en réponse à la demande, au moins un des un ou plusieurs biomarqueurs ultrasonores manquants.

6. Procédé selon la revendication 1, dans lequel la pluralité de biomarqueurs ultrasonores comprend un ou plusieurs éléments parmi la fraction d'éjection, la contrainte longitudinale globale, la vitesse de propagation de flux, la vitesse d'entrée mitrale en début de diastole, la vitesse d'entrée mitrale en fin de diastole, la vitesse annulaire mitrale en début de diastole, la vitesse annulaire mitrale en fin de diastole, l'indice de volume de l'oreillette gauche, l'épaisseur du ventricule gauche, l'épaisseur du septum, l'épaisseur d'une ou de plusieurs valvules, l'épaisseur du ventricule droit, l'épaisseur relative de la paroi, la vitesse de régurgitation tricuspide et l'indice de masse du ventricule gauche.

7. Procédé selon la revendication 1, dans lequel les informations cliniques concernant le sujet comprennent un ou plusieurs éléments parmi un type d'examen par ultrasons, une raison pour l'analyse par ultrasons, l'âge du sujet, le sexe du sujet, l'indice de masse corporelle du sujet, l'état ou le diagnostic de fibrillation auriculaire et l'état ou le diagnostic de maladie coronarienne.

8. Procédé selon la revendication 1, dans lequel la rigidité cardiaque est mesurée par un procédé de stimulation atriale, un procédé de fermeture de valvule et/ou un procédé à onde de cisaillement externe.

9. Système (200) pour fournir une probabilité visualisée d'une insuffisance cardiaque avec une fraction d'éjection préservée, HFpEF, pour un sujet, comprenant :
un modèle d'insuffisance cardiaque entraîné (263) ;
un processeur (220) configuré pour : (i) recevoir un résultat d'une analyse par ultrasons du cœur du sujet à partir d'un examen par ultrasons actuel ; (ii) extraire, du résultat reçu de l'analyse par ultrasons, une pluralité de biomarqueurs ultrasonores ; (iii) recevoir une mesure de rigidité cardiaque pour le cœur du sujet à partir de l'examen actuel et/ou à partir d'un examen par ultrasons précédent ; (iv) recevoir des informations cliniques concernant le sujet ; (v) analyser, à l'aide du modèle d'insuffisance cardiaque entraîné, la pluralité extraite de biomarqueurs ultrasonores, la mesure de rigidité cardiaque reçue et les informations cliniques reçues concernant le sujet pour générer une probabilité d'au moins une étiologie HFpEF d'une pluralité d'étiologies HFpEF, dans lequel la probabilité générée d'au moins une étiologie HFpEF d'une pluralité d'étiologies HFpEF comprend une probabilité que le sujet souffre d'une insuffisance cardiaque avec une fraction d'éjection préservée ; et (vi) générer une visualisation de la probabilité générée d'au moins une étiologie HFpEF d'une pluralité d'étiologies HFpEF ; et
une interface utilisateur (240) configurée pour fournir la probabilité générée d'au moins une étiologie HFpEF d'une pluralité d'étiologies HFpEF.

10. Système selon la revendication 9, dans lequel la pluralité d'étiologies HFpEF comprend une amylose cardiaque, une maladie coronarienne, l'hypertension, une maladie péricardique, la cardiomyopathie hypertrophique et une maladie valvulaire.

11. Système selon la revendication 9, dans lequel l'interface utilisateur est en outre configurée pour afficher : (i) le nom du sujet ; (ii) un ou plusieurs détails sur l'analyse par ultrasons ; (iii) une probabilité de chaque étiologie HFpEF d'une pluralité d'étiologies HFpEF ; et (iv) une recommandation de traitement.

12. Système selon la revendication 9, dans lequel le processeur est en outre configuré pour : déterminer qu'un ou plusieurs biomarqueurs ultrasonores sont manquants parmi la pluralité extraite de biomarqueurs ultrasonores ; (ii) générer une demande pour les un ou plusieurs biomarqueurs ultrasonores manquants ; et recevoir, en réponse à la demande, au moins un des un ou plusieurs biomarqueurs ultrasonores manquants.

13. Système selon la revendication 9, dans lequel la pluralité de biomarqueurs ultrasonores comprend un ou plusieurs éléments parmi la fraction d'éjection, la contrainte longitudinale globale, la vitesse de propagation de flux, la vitesse d'entrée mitrale en début de diastole, la vitesse d'entrée mitrale en fin de diastole, la vitesse annulaire mitrale en début de diastole, la vitesse annulaire mitrale en fin de diastole, l'indice de volume de l'oreillette gauche, l'épaisseur du ventricule gauche, l'épaisseur du septum, l'épaisseur d'une ou de plusieurs valvules, l'épaisseur du ventricule droit, l'épaisseur relative de la paroi, la vitesse de régurgitation tricuspide et l'indice de masse du ventricule gauche.

14. Système selon la revendication 9, dans lequel : (i) les informations cliniques concernant le sujet comprennent un ou plusieurs éléments parmi un type d'examen par ultrasons, une raison pour l'analyse par ultrasons, l'âge du sujet, le sexe du sujet, l'indice de masse corporelle du sujet, l'état ou le diagnostic de fibrillation auriculaire et l'état ou le diagnostic de maladie coronarienne et/ou (ii) la rigidité cardiaque est mesurée par un procédé de stimulation atriale, un procédé de fermeture de valvule et/ou un procédé à onde de cisaillement externe.

15. Support de stockage non transitoire lisible par ordinateur comprenant des instructions de code de programme informatique qui, lorsqu'elles sont exécutées par un processeur, permettent au processeur d'exécuter le procédé selon la revendication 1.
